# EUROPEAN PATENT SPECIFICATION

(11) **EP 0 604 730 B1**
(45) Date of publication and mention of the grant of the patent: **15.12.1999**
(21) Application number: 93117425.4
(22) Date of filing: 27.10.1993
(51) Int. Cl.: A61L 15/62

(54) **Hydrodisintegratable material and products formed thereby**
In Wasser desintegrierendes Material und daraus hergestellte Gegenstände
Matériau désintégrable dans l'eau et articles fabriqués à partir de ce matériau

(30) Priority: 29.12.1992 US 997797
(43) Date of publication of application: 06.07.1994
(73) Proprietor: KIMBERLY-CLARK WORLDWIDE, INC., Neenah, Wisconsin 54956 (US)
(72) Inventor: Cohen, Bernard, Berkely Lake, Georgia 30136 (US); Jameson, Lee Kirby, Roswell, Georgia 30075 (US); Isaac, Robert Lewis, Bethesda, Maryland 20817 (US)
(74) Representative: Grünecker, Kinkeldey, Stockmair & Schwanhäusser Anwaltssozietät

(56) References cited:
- GB-A- 2 048 078
- US-A- 3 952 347

## Description

The field of the present invention is that of materials which have the ability to rapidly disintegrate in an aqueous medium when subjected to agitation.

For many years the problem of disposability has plagued the industries which provide disposable diapers, incontinent garments and feminine care products. While much headway has been made in addressing this problem, one of the weak links has been the inability to create an economical plastic material which will readily dissolve or disintegrate in water. See, for example, U.K. patent disclosure 2,241,373 and U.S. Patent Number 4,186,233. Without such a product, the ability of the user to dispose of the product by flushing it down the toilet is greatly reduced if not eliminated. Furthermore, the ability of the product to disintegrate in a landfill is quite limited because a large portion of the components of the product, which may well be biodegradable or photodegradable, are encapsulated in plastic which degrades over a long period of time, if at all. Accordingly, if the plastic at least disintegrated in the presence of water, the internal components could degrade as a result of the rupture of the plastic encapsulation. GB-A-2048078 discloses a sanitary napkin having at least one layer of a water-absorbable substance containing a polymeric substance and at least one layer consisting of a film based on polyvinyl-alcohol. US-A-3952347 discloses a biodegradable barrier film comprising a non-biodegradable matrix having a biodegradable material homogeneously dispersed therein.

Accordingly, it is the object of the present invention to provide a material which readily disintegrates when in the presence of water and a thin film which readily disintegrates when agitated in the presence of water.

It should be understood that the detailed description of the presently preferred embodiment of the present invention is given only by way of illustration.

As used herein, the term "xerogellant" refers to a material which, when in a substantially dry state, has the ability to spontaneously imbibe at least about twenty (20) times its own weight in aqueous fluid. Importantly, the xerogellant should have the ability to generally retain its original identity after it has imbibed the fluid. For example, a bead, fiber or film formed from a xerogellant will still be recognizable as such after having imbibed the fluid.

As used herein, the term "water dispersible polymer" refers to a polymeric material which is capable of forming a dispersion in an aqueous medium at ambient temperature.

As used herein, the term "plasticizing agent" refers to an organic compound which, when added to a high polymer, may increase the ease of processing the high polymer or increase the toughness and flexibility of the high polymer after processing. A plasticizing agent may be able to accomplish all of these.

As used herein, the term "hydrodisintegratable" refers to a material which, in the presence of water, disintegrates into a particulate form where no individual particle is readily apparent to the unaided eye. Particles of this size generally have a maximum largest dimension of less than about one (1) millimeter.

As used herein, the term "thin film" refers to a film having an average thickness of less than about 0,254 mm (10 mils). For example, the thin film may have an average thickness of less than about 0127 mm (5 mils). More particularly, the thin film may have an average thickness of less than about 0,0254 mm (1 mil). Average thickness is determined by five (5) random measurements of the film and averaging the results.

As used herein, the term "snag test" refers to a test procedure developed and used by the National Sanitation Foundation (NSF) of Ann Arbor, Michigan, to measure the time it takes a material to disintegrate under simulated sewage conditions. The test was slightly modified by us as noted below. The test is conducted by placing an about 5,08 cm by 10,16 cm (two (2) inch by about four (4) inch) sample of plastic material on a hook shaped rod (modification-the test uses a straight rod) and stapling it to itself to form a loop to prevent it from slipping off (modification- the NSF procedure does nothing to insure the sample will stay on the rod/hook). The hook is lowered into a two (2) liter beaker of distilled water with a pH of about 7 and which is maintained at room temperature. The sample is stirred at 550 RPM. The time for the sample to break up and off the rod/hook is noted as well as the time required for the sample to disintegrate to a predetermined particle size.

The object of the present invention is solved by providing a hydrodisintegratable material comprising:
from about 7.5 to about 85 weight percent of a water dispersible polymer;
from about 7.5 to about 85 weight percent of a xerogellant; and from about 7.5 to about 20 weight percent of a plasticizing agent;
wherein the water dispersible polymer is selected from the group consisting of high molecular weight amorphous polyesters having one or more ionic substituents attached thereto and elastomeric emulsions comprising about 50% latex, about 50% water less than about 0.01% acrylamide, less than about 1.0% ammonium hydroxide, less than about 0.01% ethyl acrylate, less than about 0.1% formaldehyde and less than about 0.0025% N-methylolacrylamide.

In some embodiments the hydrodisintegratable material may include from about 15 to about 75 weight percent of a water dispersible polymer; from about 15 to about 75 weight percent of a xerogellant and from about 10 to about 15 weight percent of a plasticizing agent. For example, the hydrodisintegratable material may include from about 30 to about 60 weight percent of a water dispersible polymer; from about 30 to about 60 weight percent of a xerogellant and from about 10 to about 15 weight percent of a plasticizing agent. More particularly, the hydrodisintegratable material may include from about 40 to about 50 weight percent of a water dispersible polymer; from about 40 to about 50 weight percent of a xerogellant and about 12 weight percent of a plasticizing agent.

In some embodiments the xerogellant may be selected from the group including sodium carboxymethyl cellulose, derivatives of sodium carboxymethyl cellulose, poly(acrylic acid) salts, (ethylene oxide), acrylonitrile-grafted starch, hydrolyzed polyacrylonitrile, poly(vinyl alcohol-sodium acrylate) and polyisobutylene-co-disodium maleate.

In some embodiments the plasticizing agent may be selected from the group including glycerin, sorbitol, glucidol, sucrose, ethylene glycol, propylene glycol, diethylene glycol, polyethylene glycol, acid amides, dimethyl acetamide, dimethyl sulfoxide, methyl pyrrolidene and tetramethylene sulfone.

The present invention furthermore provides a hydrodisintegratable thin film comprising:
from about 7.5 to about 85 weight percent of a water dispersible polymer;
from about 7.5 to about 85 weight percent of a xerogellant; and
from about 7.5 to about 20 weight percent of a plasticizing agent
wherein the water dispersible polymer is selected from the group consisting of high molecular weight amorphous polyesters having one or more ionic substituents attached thereto and elastomeric emulsions comprising about 50% latex, about 50% water, less than about 0.01% acrylamide, less than about 1.0% ammonium hydroxide, less than about 0.01% ethyl acrylate, less than about 0.1% formaldehyde and less than about 0.0025% N-methylolacrylamide.

Preferably the hydrodisintegratable thin film comprises
from about 15 to about 75 weight percent of a water dispersible polymer;
from about 15 to about 75 weight percent of a xerogellant; and
from about 10 to about 15 weight percent of a plasticizing agent.

More preferably the hydrodisintegratable thin film comprises
from about 30 to about 60 weight percent of a water dispersible polymer;
from about 30 to about 60 weight percent of a xerogellant; and
from about 10 to about 15 weight percent of a plasticizing agent.

Even more preferably the hydrodisintegratable thin film comprises
from about 40 to about 50 weight percent of a water dispersible polymer;
from about 40 to about 50 weight percent of a xerogellant; and
from about 12 weight percent of a plasticizing agent.

The film is useful in the formation of disposable diapers and feminine care products which may be flushed down the toilet.

In some embodiments the thin film is capable of hydrodisintegrating, when subjected to standardized agitation testing by snag testing, in less than 25 % of the time it would take the same film formed without the xerogellant, to disintegrate, if it disintegrates at all. More particularly, the thin film may be capable of hydrodisintegrating, when subjected to standardized agitation testing by snag testing, in less than 10% of the time it would take the same film formed without the xerogellant, to disintegrate, if it disintegrates at all.

The xerogellant is selected preferably from the group consisting of sodium carboxymethyl cellulose, derivatives of sodium carboxymethyl cellulose, poly(acrylic acid) salts, poly(ethylene oxide), acrylonitrile-grafted starch, hydrolyzed polyacrylonitrile, poly(vinyl alcohol-sodium acrylate), polyisobutylene-co-disodium maleate.

Preferably the plasticizing agent is selected from the group consisting of glycerin, sorbitol, glucidol, sucrose, ethylene glycol, propylene glycol, diethylene glycol, polyethylene glycol, acid amide, dimethyl acetamide, dimethyl sulfoxide, methyl pyrrolidene and tetramethylene sulfone.

The hydrodisintegratable material of the present invention is formed by placing the xerogellant, preferably in powder form, in an appropriately sized container and adding water so that the xerogellant is fully hydrated. While any material meeting the definition of a xerogellant may be utilized, exemplary xerogellants include sodium carboxymethyl cellulose, derivatives of sodium carboxymethyl cellulose, poly(acrylic acid) salts, (ethylene oxide), acrylonitrilegrafted starch, hydrolyzed polyacrylonitrile, poly(vinyl alcohol-sodium acrylate) and polyisobutylene-co-disodium maleate. One xerogellant is a starch grafted sodium polyacrylate which may be obtained from Hoechst Celanese Corporation under the trade designation Sanwet IM5000P.

If the consistency of the xerogellant and water mixture is not that of a fluid, additional water is added until such is the case. This action is only necessary with certain xerogellants. It is to the fluidized xerogellant that the water dispersible polymer and the plasticizing agent are added. The water dispersible polymer may be added to the hydrated xerogellant as an aqueous dispersion.

While any film forming water dispersible polymer may be utilized, exemplary film forming water dispersible polymers include such polymers chosen from the group including high molecular weight amorphous polyesters having one or more ionic substituents attached thereto. This type of polymer is available form the Eastman Kodak Co. of Rochester, N.Y. under the trade designation Eastman AQ. In particular, Eastman AQ 55D and AQ 38D. Alternatively, the water dispersible polymer may be selected from the group including elastomeric emulsions, acrylic polymers, polyoxides, vinyl polymers, cellulose derivatives, starch derivatives, polysaccahrides, proteins and copolymers thereof. Exemplary elastomeric emulsions may be obtained from the B.F. Goodrich Co., Specialty Polymers & Chemicals Division under the trade designation HyStretch. HyStretch elastomeric emulsions typically are a blend of about 50% latex, about 50% water, less than about 0.01 % acrylamide, less than about 1.0% ammonium hydroxide, less than about 0.01 % ethyl acrylate, less than about 0.1 % formaldehyde and less than about 0.0025% N-methylolacrylamide.

While any suitable plasticizing agent may be utilized, exemplary plasticizing agents include glycerin, sorbitol, glucidol, sucrose, ethylene glycol, propylene glycol, diethylene glycol, polyethylene glycol, acid amides, dimethyl acetamide, dimethyl sulfoxide, methyl pyrrolidene and tetramethylene sulfone. One exemplary plasticizing agent is glycerin which may be obtained from Fischer Scientific of Fairtown, New Jersey, under the trade designation G-33-1.

After the three components have been thoroughly mixed, they are cast, in conventional manner, in a mold of the desired configuration of the final product desired. Thereafter the water is removed by natural evaporation which, if desired, may be assisted by low grade heating of the cast mixture. In one embodiment of the present invention the three components are cast into a thin film material.

Discounting the water which is evaporated away, the xerogellant, the film forming water dispersible polymer and the plasticizing agent are blended together in a conventional manner so that the final weight percentage of these components, after removal of the water by evaporation, is from about 7.5 to about 85 weight percent of the water dispersible polymer; from about 7.5 to about 85 weight percent of the xerogellant; and from about 7.5 to about 20 weight percent of the plasticizing agent. More particularly, the final weight percentages of these components of the material may range from about 15 to about 75 weight percent of the water dispersible polymer; from about 15 to about 75 weight percent of the xerogellant; and from about 10 to about 15 weight percent of the plasticizing agent. Even more particularly, the final weight percentages of these components may range from about 30 to about 60 weight percent of the water dispersible polymer; from about 30 to about 60 weight percent of the xerogellant; and from about 10 to about 15 weight percent of the plasticizing agent. Yet even more particularly, the final weight percentages of these components may range from about 40 to about 50 weight percent of a water dispersible polymer; from about 40 to about 50 weight percent of a xerogellant; and about 12 weight percent of the plasticizing agent.

In some embodiments it may be desirable to employ various additives such as antioxidants, antistatic agents, blowing agents, compatibilizers, flame retardants, heat stabilizers, impact modifiers, lubricants, ultraviolet stabilizers, processing aids, surfactants, dispersants, slip agents, etc., as fabricating agents or as modifiers depending on the specific properties which would be desirable to have in the final product.

The use of surfactants can further enhance the rate of hydrodisintegration of the film. Exemplary surfactants which can be utilized in the invention are (1) anionic surfactants such as carboxylic acids and salts, sulfonic acids and salts, sulfuric acid esters and salts, phosphoric and polyphosphoric acid esters and salts; (2) non-ionic surfactants such as ethoxylated alcohols, ethoxylated alhylphenols, ethoxylated carboxylic esters and ethoxylated carboxylic amides; (3) cationic surfactants such as oxygen free amines, oxygen containing amines, amide linked amines and quaternary ammonium salts: and (4) amphoteric surfactants such as imidazolinium derivatives, amino acids and their derivatives in which the nitrogen atom is protonated and alkylketaimes.

The surfactants may be added so that they form from at least about 0.01 to about 0.10 weight percent of the hydrodisintegratable film. For example, the surfactants may form from at least about 0.03 to about 0.08 weight percent of the hydrodisintegratable film. More particularly, the surfactants may form from at least about 0.05 to about 0.06 weight percent of the hydrodisintegratable film.

Those of skill in the art will readily recognize that the hydrodisintegratable material may be formed by other methods. For example, the material may be formed by extrusion methods as demonstrated in Example III, below.

The invention will now be described with respect to certain specific embodiments thereof.

### EXAMPLE I

A number of film samples were prepared where the xerogellant was a starch grafted sodium polyacrylate obtained from Hoechst Celanese Corporation under the trade designation Sanwet IM5000P, the water dispersible polymer was a high molecular weight amorphous polyester having one or more ionic substituents attached thereto which was obtained from the Eastman Kodak Co. under the trade designation AQ 55D, and the plasticizer was glycerin obtained from Fisher Scientific under the trade designation G-33-1. The samples represented a wide range of variation in the amount of xerogellant present. Snag testing was done on all of the samples and the snag test results of the samples were compared to a film formed without the xerogellant being present. Generally speaking, the snag testing demonstrated that the larger the weight percentage of xerogellant present, the quicker the film began to disintegrate.

The films were formed by combining the appropriate amount (See Table I.) of the xerogellant with 600 milliliters of water is an 800 milliliter beaker. The solution was stirred gently and the xerogellant was allowed to hydrate for thirty (30) minutes. The hydrated xerogellant was then poured into a Waring Blender and liquified by being mixed at low speed for two (2) minutes. The liquified solution was poured back into the beaker and the appropriate amount of AQ 55D and glycerol was then added. The mixture was stirred by hand gently with a glass stir rod and then mechanically with a magnetic stir bar until thoroughly mixed. Then the mixture was poured into individual (2.5 inch by 11 inch by 0.05 inch) wax molds manufactured by McKellco of Alpharetta, Georgia. The solutions evaporated in seven (7) days. The dried films were gently removed from each of the wax molds and cut into two (2) inch by four (4) inch strips for snag testing and evaluation.

While those in the art will be readily able to determine the actual amounts of materials used in each sample, the amounts, for the 43.5/43.5/13 sample were 2.8 grams of Sanwet 1M5000P, 10 grams of AQ 55D solution and 0.84 grams of glycerol.

The results of these tests are reported below in Table I.

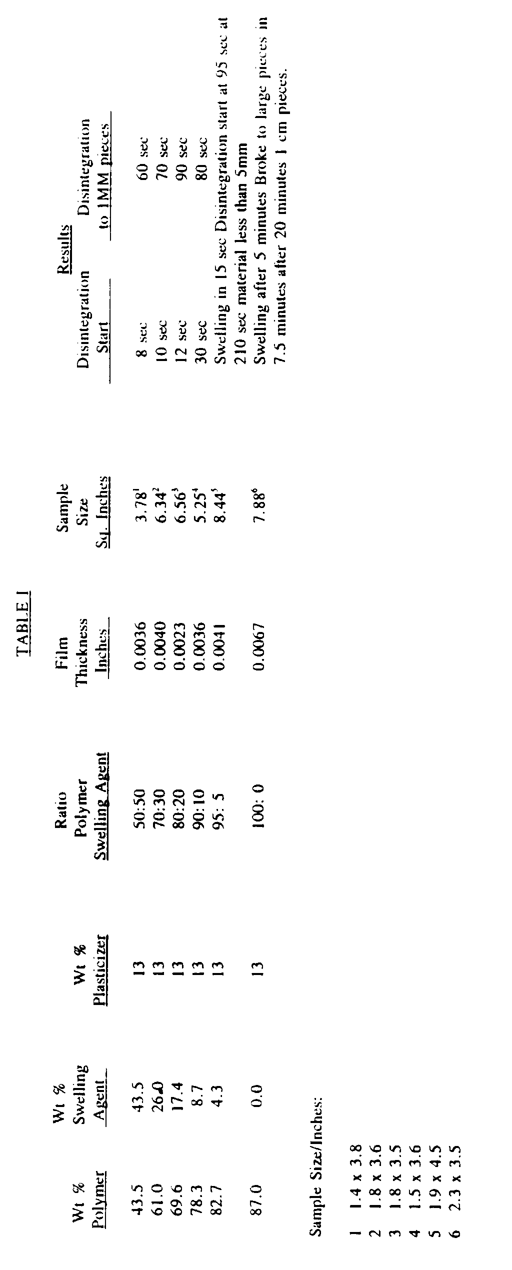

### EXAMPLE II

Example I was repeated with the exception that the high molecular weight amorphous polyester was obtained from the Eastman Kodak Co. under the trade designation AQ 38D.

The results of these tests are reported below in Table II.

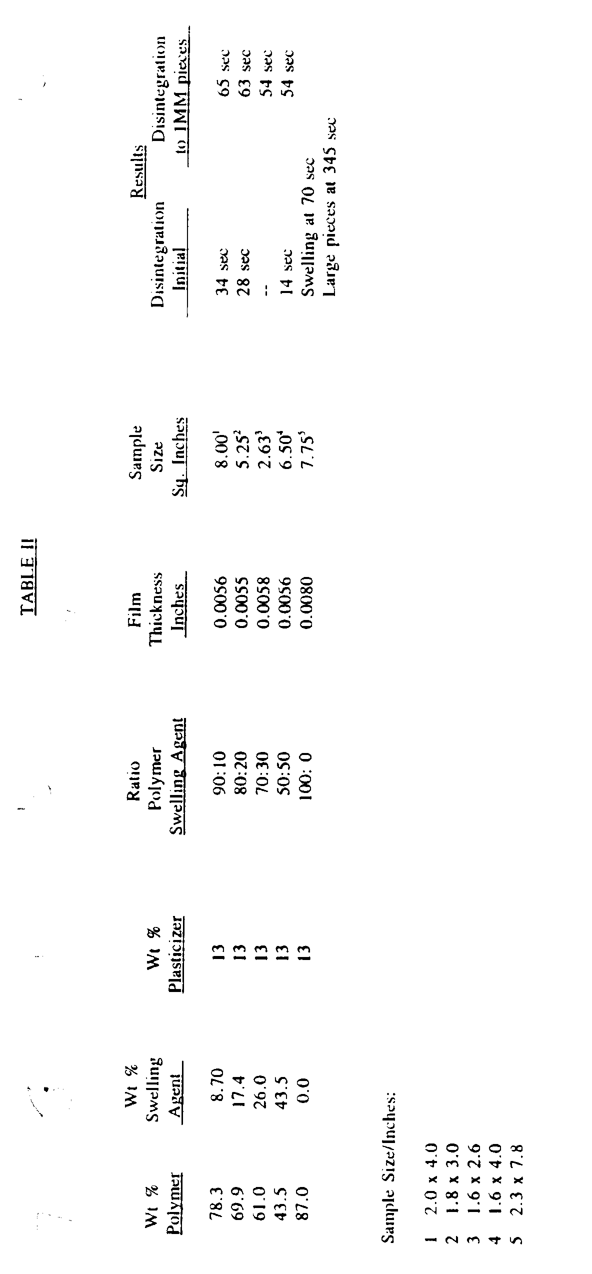

### EXAMPLE III

This example demonstrated that the hydrodisintegratable material may be formed by film extrusion processes as compared to Examples I and II where the material was formed by casting.

1,105 milliliters of 96% glycerin (USP grade), obtained from the Dow Chemical Co. of Midland, Michigan, was combined with 1,000 milliliters of water to produce a 11.52% glycerin solution. This solution was mixed thoroughly and then pumped by a Neptune Proportioning Pump, model no. 520-A-N3, (Neptune Chemical Pump Co., Lansdale, PA) into a twin screw extruder, provided with a polymer and powder feed, purchased from Werner & Pfleiderer Corp. of Ramsey, New Jersey. The pump was adjusted until the solution feed rate of 125 grams per minute was obtained (setting 95). Eastman AQ 38S was added to the pellet hopper of the extruder and Sanwet IM5000P xerogellant was added to the powder hopper of the extruder. The extrusion rate was adjusted until feed rates of 50 grams per minute were obtained for both the water dispersible AQ 38S and the xerogellant. (Pellet setting of 29 and a powder setting of 180.) The die was removed from the end of the extruder to allow for easier sample flow from the end of the barrel. The sample was extruded as a 1.5 inch wide strip. At the time of sample collection, the following extruder conditions were recorded.

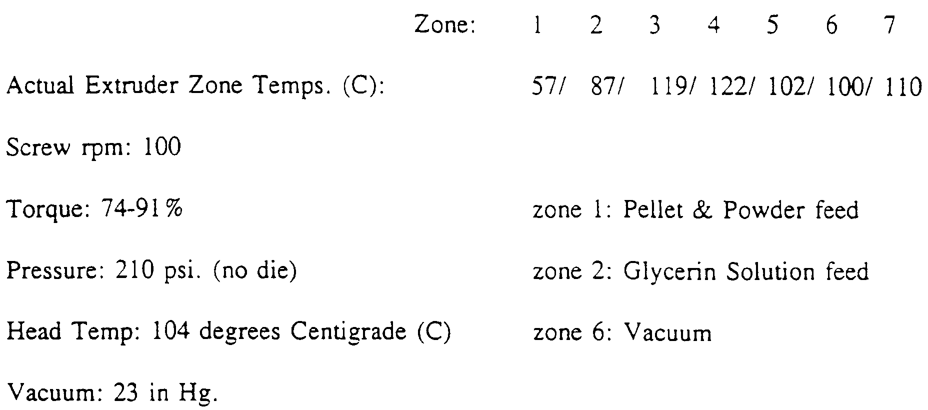

The material formed by this method was also readily hydrodisintegratable.

## Claims

1. A hydrodisintegratable material comprising:
from about 7.5 to about 85 weight percent of a water dispersible polymer;
from about 7.5 to about 85 weight percent of a xerogellant; and
from about 7.5 to about 20 weight percent of a plasticizing agent;
wherein the water dispersible polymer is selected from the group consisting of high molecular weight amorphous polyesters having one or more ionic substituents attached thereto and elastomeric emulsions comprising about 50% latex, about 50% water, less than about 0.01% acrylamide, less than about 1.0% ammonium hydroxide, less than about 0.01% ethyl acrylate, less than about 0.1% formaldehyde and less than about 0.0025% N-methylolacrylamide.

2. The hydrodisintegratable material of claim 1, comprising:
from about 15 to about 75 weight percent of a water dispersible polymer;
from about 15 to about 75 weight percent of a xerogellant; and
from about 10 to about 15 weight percent of a plasticizing agent.

3. The hydrodisintegratable material of claim 1, comprising:
from about 30 to about 60 weight percent of a water dispersible polymer;
from about 30 to about 60 weight percent of a xerogellant; and
from about 10 to about 15 weight percent of a plasticizing agent.

4. The hydrodisintegratable material of claim 1, comprising:
from about 40 to about 50 weight percent of a water dispersible polymer;
from about 40 to about 50 weight percent of a xerogellant; and
from about 12 weight percent of a plasticizing agent.

5. The hydrodisintegratable material of claim 1 wherein the xerogellant is selected from the group consisting of sodium carboxymethyl cellulose, derivatives of sodium carboxymethyl cellulose, poly(acrylic acid) salts, poly(ethylene oxide), acrylonitrile-grafted starch, hydrolyzed polyacrylonitrile, poly(vinyl alcohol-sodium acrylate), polyisobutylene-co-disodium maleate.

6. The hydrodisintegratable material of claim 1, wherein the plasticizing agent is selected from the group consisting of glycerin, sorbitol, glucidol, sucrose, ethylene glycol, propylene glycol, diethylene glycol, polyethylene glycol, acid amide, dimethyl acetamide, dimethyl sulfoxide, methyl pyrrolidene and tetramethylene sulfone.

7. A hydrodisintegratable thin film comprising:
from about 7.5 to about 85 weight percent of a water dispersible polymer;
from about 7.5 to about 85 weight percent of a xerogellant; and
from about 7.5 to about 20 weight percent of a plasticizing agent
wherein the water dispersible polymer is selected from the group consisting of high molecular weight amorphous polyesters having one or more ionic substituents attached thereto and elastomeric emulsions comprising about 50% latex, about 50% water, less than about 0.01% acrylamide, less than about 1.0% ammonium hydroxide, less than about 0.01% ethyl acrylate, less than about 0.1% formaldehyde and less than about 0.0025% N-methylolacrylamide.

8. The hydrodisintegratable thin film of claim 7, comprising:
from about 15 to about 75 weight percent of a water dispersible polymer;
from about 15 to about 75 weight percent of a xerogellant; and
from about 10 to about 15 weight percent of a plasticizing agent.

9. The hydrodisintegratable thin film of claim 7, comprising:
from about 30 to about 60 weight percent of a water dispersible polymer;
from about 30 to about 60 weight percent of a xerogellant; and
from about 10 to about 15 weight percent of a plasticizing agent.

10. The hydrodisintegratable thin film of claim 7, comprising:
from about 40 to about 50 weight percent of a water dispersible polymer;
from about 40 to about 50 weight percent of a xerogellant; and
from about 12 weight percent of a plasticizing agent.

11. The hydrodisintegratable thin film of claim 7, wherein the film is adapted to disintegrate into particles having a maximum diameter of less than about 1 millimeter, when subjected to a snag test, in less than 25% of the time that it would take a like film without the xerogellant to so disintegrate.

12. The hydrodisintegratable thin film of claim 7, wherein the film is adapted to disintegrate into particles having a maximum diameter of less than about 1 millimeter, when subjected to a snag test, in less than 10% of the time that it would take a like film without the xerogellant to so disintegrate.

13. The hydrodisintegratable thin film of claim 7 wherein the xerogellant is selected from the group consisting of sodium carboxymethyl cellulose, derivatives of sodium carboxymethyl cellulose, poly(acrylic acid) salts, poly(ethylene oxide), acrylonitrile-grafted starch, hydrolyzed polyacrylonitrile, poly(vinyl alcohol-sodium acrylate), polyisobutylene-co-disodium maleate.

14. The hydrodisintegratable thin film of claim 7 wherein the plasticizing agent is selected from the group consisting of glycerin, sorbitol, glucidol, sucrose, ethylene glycol, propylene glycol, diethylene glycol, polyethylene glycol, acid amide, dimethyl acetamide, dimethyl sulfoxide, methyl pyrrolidene and tetramethylene sulfone.

## Patentansprüche

1. In Wasser zerfallendes Material, umfassend:
etwa 7,5 bis etwa 85 Gewichtsprozent eines wasserdispergierbaren Polymeren;
etwa 7,5 bis etwa 85 Gewichtsprozent eines xerogelierenden Mittels; und
etwa 7,5 bis etwa 20 Gewichtsprozent eines Weichmachers;
worin das wasserdispergierbare Polymer ausgewählt ist aus der Gruppe bestehend aus amorphen Polyestern mit hohem Molekulargewicht mit einem oder mehreren daran gebundenen ionischen Substituenten und Elastomer-Emulsionen, die etwa 50% Latex, etwa 50% Wasser, weniger als etwa 0,01% Acrylamid, weniger als etwa 1,0% Ammoniumhydroxid, weniger als etwa 0,01% Ethylacrylat, weniger als etwa 0,1% Formaldehyd und weniger als etwa 0,0025% N-Methylolacrylamid umfassen.

2. In Wasser zerfallendes Material von Anspruch 1, umfassend:
etwa 15 bis etwa 75 Gewichtsprozent eines wasserdispergierbaren Polymeren;
etwa 15 bis etwa 75 Gewichtsprozent eines xerogelierenden Mittels; und
etwa 10 bis etwa 15 Gewichtsprozent eines Weichmachers.

3. In Wasser zerfallendes Material von Anspruch 1, umfassend:
etwa 30 bis etwa 60 Gewichtsprozent eines wasserdispergierbaren Polymeren;
etwa 30 bis etwa 60 Gewichtsprozent eines xerogelierenden Mittels; und
etwa 10 bis etwa 15 Gewichtsprozent eines Weichmachers.

4. In Wasserzerfallendes Material von Anspruch 1, umfassend:
etwa 40 bis etwa 50 Gewichtsprozent eines wasserdispergierbaren Polymeren;
etwa 40 bis etwa 50 Gewichtsprozent eines xerogelierenden Mittels; und
etwa 12 Gewichtsprozent eines Weichmachers.

5. In Wasser zerfallendes Material von Anspruch 1, worin das xerogelierende Mittel aus der aus Natrium-Carboxymethylcellulose, Derivaten von Natrium-Carboxymethylcellulose, Polyacrylsäuresalzen, Polyethylenoxid, Acrylnitril-gepfropfter Stärke, hydrolysiertem Polyacrylnitril, Poly(vinylalkohol-Natriumacrylat), Polyisobutylen-Dinatriummaleat-Copolymerisat bestehenden Gruppe ausgewählt ist.

6. In Wasser zerfallendes Material von Anspruch 1, worin der Weichmacher aus der aus Glycerin, Sorbit, Glucit, Sucrose, Ethylenglykol, Propylenglykol, Diethylenglykol, Polyethylenglykol, Säureamid, Dimethylacetamid, Dimethylsulfoxid, Methylpyrroliden und Tetramethylensulfon bestehenden Gruppe ausgewählt ist.

7. In Wasser zerfallender dünner Film, umfassend:
etwa 7,5 bis etwa 85 Gewichtsprozent eines wasserdispergierbaren Polymeren;
etwa 7,5 bis etwa 85 Gewichtsprozent eines xerogelierenden Mittels; und
etwa 7,5 bis etwa 20 Gewichtsprozent eines Weichmachers;
worin das wasserdispergierbare Polymer ausgewählt ist aus der Gruppe bestehend aus amorphen Polyestern mit hohem Molekulargewicht mit einem oder mehreren daran gebundenen ionischen Substituenten und Elastomer-Emulsionen, die etwa 50% Latex, etwa 50% Wasser, weniger als etwa 0,01% Acrylamid, weniger als etwa 1,0% Ammoniumhydroxid, weniger als etwa 0,01% Ethylacrylat, weniger als etwa 0,1% Formaldehyd und weniger als etwa 0,0025% N-Methylolacrylamid umfassen.

8. In Wasser zerfallender dünner Film von Anspruch 7, umfassend:
etwa 15 bis etwa 75 Gewichtsprozent eines wasserdispergierbaren Polymeren;
etwa 15 bis etwa 75 Gewichtsprozent eines xerogelierenden Mittels; und
etwa 10 bis etwa 15 Gewichtsprozent eines Weichmachers.

9. In Wasser zerfallender dünner Film von Anspruch 7, umfassend:
etwa 30 bis etwa 60 Gewichtsprozent eines wasserdispergierbaren Polymeren;
etwa 30 bis etwa 60 Gewichtsprozent eines xerogelierenden Mittels; und
etwa 10 bis etwa 15 Gewichtsprozent eines Weichmachers.

10. In Wasser zerfallender dünner Film von Anspruch 7, umfassend:
etwa 40 bis etwa 50 Gewichtsprozent eines wasserdispergierbaren Polymeren;
etwa 40 bis etwa 50 Gewichtsprozent eines xerogelierenden Mittels; und
etwa 12 Gewichtsprozent eines Weichmachers.

11. In Wasser zerfallender dünner Film von Anspruch 7, wobei der Film für den Zerfall in Partikel mit einem maximalen Durchmesser von weniger als etwa 1 mm so adaptiert ist, daß in einem Hakentest weniger als 25% der Zeit benötigt wird, die zu einem solchen Zerfall bei einem ähnlichen Film ohne xerogelierendes Mittel benötigt würde.

12. In Wasser zerfallender dünner Film von Anspruch 7, wobei der Film für den Zerfall in Partikel mit einem maximalen Durchmesser von weniger als etwa 1 mm so adaptiert ist, daß in einem Hakentest weniger als 10% der Zeit benötigt wird, die zu einem solchen Zerfall bei einem ähnlichen Film ohne xerogelierendes Mittel benötigt würde.

13. In Wasser zerfallender dünner Film von Anspruch 7, worin das xerogelierende Mittel aus der aus Natrium-Carboxymethylcellulose, Derivaten von Natrium-Carboxymethylcellulose, Polyacrylsäuresalzen, Polyethylenoxid, Acrylnitrilgepfropfter Stärke, hydrolysiertem Polyacrylnitril, Poly(vinylalkohol-Natriumacrylat), Polyisobutylen-Dinatriummaleat-Copolymerisat bestehenden Gruppe ausgewählt ist.

14. In Wasser zerfallender dünner Film von Anspruch 7, worin der Weichmacher aus der aus Glycerin, Sorbit, Glucit, Sucrose, Ethylenglykol, Propylenglykol, Diethylenglykol, Polyethylenglykol, Säureamid, Dimethylacetamid, Dimethylsulfoxid, Methylpyrroliden und Tetramethylensulfon bestehenden Gruppe ausgewählt ist.

## Revendications

1. Matériau susceptible de se désintégrer dans l'eau comprenant :
environ 7,5 à environ 85 pour-cent en poids d'un polymère susceptible de se disperser dans l'eau ;
environ 7,5 à environ 85 pour-cent en poids d'un xérogel ; et
environ 7,5 à environ 20 pour-cent en poids d'un agent plastifiant ;
dans lequel le polymère susceptible de se disperser dans l'eau est choisi parmi le groupe constitué de polyesters amorphes de poids moléculaire élevé ayant un ou plusieurs substituants ioniques qui leur sont attachés, et des émulsions élastomères comprenant environ 50% de latex, environ 50% d'eau, moins d'environ 0,01% d'acrylamide, moins d'environ 1,0% d'hydroxyde d'ammonium, moins d'environ 0,01% d'acrylate d'éthyle, moins d'environ 0,1% de formaldéhyde et moins d'environ 0,0025% de N-méthylolacrylamide.

2. Matériau susceptible de se désintégrer dans l'eau de la revendication 1, comprenant :
environ 15 à environ 75 pour-cent en poids d'un polymère susceptible de se disperser dans l'eau ;
environ 15 à environ 75 pour-cent en poids d'un xérogel ; et
environ 10 à environ 15 pour-cent en poids d'un agent plastifiant.

3. Matériau susceptible de se désintégrer dans l'eau de la revendication 1, comprenant :
environ 30 à environ 60 pour-cent en poids d'un polymère susceptible de se disperser dans l'eau ;
environ 30 à environ 60 pour-cent en poids d'un xérogel ; et
environ 10 à environ 15 pour-cent en poids d'un agent plastifiant.

4. Matériau susceptible de se désintégrer dans l'eau de la revendication 1, comprenant :
environ 40 à environ 50 pour-cent en poids d'un polymère susceptible de se disperser dans l'eau ;
environ 40 à environ 50 pour-cent en poids d'un xérogel ; et
environ 12 pour-cent en poids d'un agent plastifiant.

5. Matériau susceptible de se désintégrer dans l'eau de la revendication 1, dans lequel le xérogel est choisi parmi le groupe constitué de la carboxyméthylcellulose de sodium, des dérivés de carboxyméthylcellulose de sodium, des sels d'acide poly(acrylique), du poly(éthylène oxyde), de l'amidon greffé avec de l'acrylonitrile, du polyacrylonitrile hydrolysé, du poly(alcool vinylique-acrylate de sodium), du polyisobutylène-co-maléate de disodium.

6. Matériau susceptible de se désintégrer dans l'eau de la revendication 1, dans lequel l'agent plastifiant est choisi parmi le groupe constitué de la glycérine, du sorbitol, du glucidol, du saccharose, de l'éthylèneglycol, du propylèneglycol, du diéthylèneglycol, du polyéthylèneglycol, de l'amide acide, du diméthylacétamide, du sulfoxyde de diméthyle, du méthylpyrrolidène et de la tétraméthylènesulfone.

7. Film mince susceptible de se désintégrer dans l'eau comprenant :
environ 7,5 à environ 85 pour-cent en poids d'un polymère susceptible de se disperser dans l'eau ;
environ 7,5 à environ 85 pour-cent en poids d'un xérogel ; et
environ 7,5 à environ 20 pour-cent en poids d'un agent plastifiant ;
dans lequel le polymère susceptible de se disperser dans l'eau est choisi parmi le groupe constitué de polyesters amorphes de poids moléculaire élevé ayant un ou plusieurs substituants ioniques qui leur sont attachés, et des émulsions élastomères comprenant environ 50% de latex, environ 50% d'eau, moins d'environ 0,01% d'acrylamide, moins d'environ 1,0% d'hydroxyde d'ammonium, moins d'environ 0,01% d'acrylate d'éthyle, moins d'environ 0,1% de formaldéhyde et moins d'environ 0,0025% de N-méthylolacrylamide.

8. Film mince susceptible de se désintégrer dans l'eau de la revendication 7, comprenant :
environ 15 à environ 75 pour-cent en poids d'un polymère susceptible de se disperser dans l'eau ;
environ 15 à environ 75 pour-cent en poids d'un xérogel ; et
environ 10 à environ 15 pour-cent en poids d'un agent plastifiant.

9. Film mince susceptible de se désintégrer dans l'eau de la revendication 7, comprenant :
environ 30 à environ 60 pour-cent en poids d'un polymère susceptible de se disperser dans l'eau ;
environ 30 à environ 60 pour-cent en poids d'un xérogel ; et
environ 10 à environ 15 pour-cent en poids d'un agent plastifiant.

10. Film mince susceptible de se désintégrer dans l'eau de la revendication 7, comprenant :
environ 40 à environ 50 pour-cent en poids d'un polymère susceptible de se disperser dans l'eau ;
environ 40 à environ 50 pour-cent en poids d'un xérogel ; et
environ 12 pour-cent en poids d'un agent plastifiant.

11. Film mince susceptible de se désintégrer dans l'eau de la revendication 7, dans lequel le film est conçu pour se désintégrer en particules ayant un diamètre maximum inférieur à environ l millimètre, lorsqu'il est soumis à un test de résistance à l'accroc, en moins de 25% du temps qu'un film semblable sans le xérogel mettrait à se désintégrer.

12. Film mince susceptible de se désintégrer dans l'eau de la revendication 7, dans lequel le film est conçu pour se désintégrer en particules ayant un diamètre maximum inférieur à environ l millimètre, lorsqu'il est soumis à un test de résistance à l'accroc, en moins de 10% du temps qu'un film semblable sans le xérogel mettrait à se désintégrer.

13. Film mince susceptible de se désintégrer dans l'eau de la revendication 7, dans lequel le xérogel est choisi parmi le groupe constitué de la carboxyméthylcellulose de sodium, des dérivés de carboxyméthylcellulose de sodium, des sels d'acide poly(acrylique), du poly(éthylène oxyde), de l'amidon greffé avec de l'acrylonitrile, du polyacrylonitrile hydrolysé, du poly(alcool vinylique-acrylate de sodium), du polyisobutylène-co-maléate de disodium.

14. Film mince susceptible de se désintégrer dans l'eau de la revendication 7, dans lequel l'agent plastifiant est choisi parmi le groupe constitué de la glycérine, du sorbitol, du glucidol, du saccharose, de l'éthylèneglycol, du propylèneglycol, du diéthylèneglycol, du polyéthylèneglycol, de l'amide acide, de diméthylacétamide, du sulfoxyde de diméthyle, du méthylpyrrolidène et de la tétraméthylènesulfone.
